(19) Europäisches Patentamt
European Patent Office
Office européen des brevets

(11) EP 1 820 492 B1

(12) FASCICULE DE BREVET EUROPEEN

(45) Date de publication et mention de la délivrance du brevet:
**03.12.2008 Bulletin 2008/49**

(51) Int Cl.:
***A61K 8/97*** *(2006.01)* ***A61K 8/49*** *(2006.01)*
***A61Q 19/00*** *(2006.01)* ***A61Q 19/08*** *(2006.01)*

(21) Numéro de dépôt: **07101617.4**

(22) Date de dépôt: **02.02.2007**

(54) **Composition protectrice et régénérante**

Schützende und regenerierende Zusammensetzung

Protecting, regenerating composition

(84) Etats contractants désignés:
**DE FR GB IT**

(30) Priorité: **03.02.2006 FR 0650400**

(43) Date de publication de la demande:
**22.08.2007 Bulletin 2007/34**

(73) Titulaire: **LVMH RECHERCHE**
**45800 St. Jean de Braye (FR)**

(72) Inventeurs:
- **Andre, Patrice**
**45170 Neuville aux bois (FR)**
- **Renimel, Isabelle**
**45470 Trainou (FR)**

(74) Mandataire: **Portal, Gérard et al**
**Cabinet Beau de Loménie**
**158, rue de l'Université**
**75340 Paris Cédex 07 (FR)**

(56) Documents cités:
**DE-A1- 19 933 464**
**FR-A- 2 844 715**
**US-A1- 2005 201 955**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne une composition protectrice et régénérante.

La présente invention concerne en particulier une composition comprenant une association d'un premier ingrédient cosmétiquement actif comprenant un extrait de sarment de vigne et d'un deuxième ingrédient cosmétiquement actif comprenant un composant de type ectoine. Plus particulièrement, ledit deuxième ingrédient est choisi parmi l'ectoine, l'hydroxyectoine et leurs mélanges.

La présente invention concerne plus particulièrement une composition cosmétique d'application topique sur la peau comprenant une telle composition.

L'invention concerne aussi un procédé de soin cosmétique comprenant l'application topique sur la peau d'une personne voulant réaliser un soin cosmétique avec cette composition.

### ETAT DE LA TECHNIQUE

Il est connu, par le document WO 01/03713, un procédé d'extraction de resvératrol et/ou de viniférine à partir de sarments de vigne secs. Il est rappelé que la viniférine est le dimère du resvératrol. Ce document indique aussi que le resvératrol se présente généralement sous la forme monomère, mais existe également comme oligomère comportant jusqu'à 4 monomères (page 1, lignes 11 à 17).

Il est souligné l'intérêt d'utiliser des sarments secs car ils présentent un rendement en resvératrol et en viniférine bien supérieur à celui obtenu avec des sarments frais (page 2, ligne 31 à page 3, ligne 2).

Ce document prévoit la possibilité d'obtenir des extraits de la vigne de vin mille fois plus concentrés en resvératrol que les extraits commercialisés et obtenus à partir de la vigne en permettant la préparation de formulation liquide, en poudre ou en comprimé ayant des teneurs élevées en resvératrol, compatibles avec des applications industrielles habituelles pour ces produits et cite à ce propos, de manière générale, les domaines pharmaceutique, diététique et cosmétique (page 5, lignes 25 à 32 et revendication 18).

Par ailleurs, il est à observer que le séchage des sarments de vigne décrit dans ce document antérieur est réalisé à l'air libre, mais pendant une période de temps n'excédant pas 4 mois (exemple 1 : 4 mois de séchage ; exemple 2 : 3 mois de séchage ; exemple 3 : le séchage le plus long est de 3 mois).

### BUTS DE L'INVENTION

La présente invention a pour but principal de fournir une nouvelle composition comprenant une association synergique d'ingrédients actifs, et plus particulièrement une composition cosmétique à application topique sur la peau.

La présente invention a encore pour but principal de fournir une telle composition à partir d'ingrédients actifs cosmétiquement acceptables, c'est-à-dire n'ayant pas de toxicité décelable sur les cellules de la peau, et disponible(s) en quantité importante à un prix raisonnable pour une utilisation dans le cadre de la fabrication de composition cosmétique à l'échelle industrielle cosmétique.

La présente invention a encore comme autre but principal de fournir une composition de teneur limitée en resvératrol au profit de ses oligomères,

### RESUME DE L'INVENTION

La présente invention résout simultanément les buts ci-dessus d'une manière simple, aisée à mettre en oeuvre à l'échelle industrielle cosmétique.

Ainsi, selon un premier aspect, la présente invention fournit une composition caractérisée en ce qu'elle comprend une association d'un premier ingrédient cosmétiquement actif comprenant un extrait de sarment séché de vigne et d'un deuxième ingrédient cosmétiquement actif comprenant un composant de type ectoine.

Dans le cadre de l'invention, on entend par l'expression "un composant de type ectoine" un acide (S)-1,4,5,6-tetrahydro-4-pyrimidine carboxylique, substitué ou non par au moins un radical alkyle inférieur en C1-C6, notamment en position 2, et/ou par au moins un groupe hydroxy ou methoxy, notamment en position 5, ainsi que ses sels ou esters cosmétiquement acceptables.

De préférence, ledit deuxième ingrédient est choisi parmi l'ectoine, ou acide (S)-1,4,5,6-tetrahydro-2-méthyl-4 pyrimidine carboxylique, et l'hydroxyectoine ou acide (S, S)-1,4,5,6-tetrahydro-5-hydroxy-2-méthyl-4-pyrimidine carboxylique, ainsi que leurs sels ou esters cosmétiquement acceptables.

Selon encore un mode de réalisation particulier de l'invention, cette composition comprend de 0,1 % à 20 % en poids, de préférence de 0,1 % à 10 % en poids du premier ingrédient, et de 0,1 % à 20 % en poids, de préférence de 0,1 % à 10 % en poids du deuxième ingrédient, le total de ces deux ingrédients étant toutefois avantageusement inférieur ou égal à 20 % en poids, de préférence inférieur à 10 % en poids, par rapport au poids total de la composition.

Selon encore un autre mode de réalisation particulier de l'invention, la proportion respective en poids entre le premier ingrédient et le deuxième ingrédient est comprise entre 1/10 et 10/1, en particulier elle est d'environ 1/1.

Selon un mode particulier de l'invention, ledit deuxième ingrédient est un mélange en proportions sensiblement égales en poids de l'ectoine et de l'hydroxyectoine.

Selon un deuxième aspect, la présente invention concerne aussi une composition cosmétique d'application topique sur la peau, caractérisée en ce qu'elle comprend une composition telle que précédemment définie ou telle que résultant de la description suivante, associée à un excipient cosmétiquement acceptable.

Selon une autre variante de réalisation, la composition est caractérisée en ce qu'elle comprend au moins un autre ingrédient cosmétiquement actif, en particulier un autre ingrédient cosmétiquement actif ayant une activité anti-vieillissement, en particulier pour prévenir, corriger ou ralentir les effets du vieillissement de la peau; par exemple la vitamine A, vitamine E, vitamine C; ou réalisant un soin de revitalisation de la peau, par exemple un madécassoside; ou un ingrédient cosmétiquement actif, ayant un effet hydratant, par exemple un ecdystéroïde ou un extrait de plante en contenant, tel qu'un extrait de Ajuga turkestanica; ou un ingrédient cosmétiquement actif de protection contre les rayonnements actiniques, par exemple un filtre solaire physique ou chimique cosmétiquement acceptable, par exemple un méthoxycinnamate.

Selon un troisième aspect, la présente invention concerne encore un procédé de soin cosmétique, caractérisé en ce qu'il comprend l'application topique sur la peau d'une personne voulant réaliser un soin cosmétique, d'une composition telle que précédemment définie ou d'une composition cosmétique telle que précédemment définie, ou telle que résultant de la description suivante.

Selon une variante de réalisation particulière du procédé, celui-ci est caractérisé en ce qu'il s'agit d'un soin cosmétique choisi parmi le groupe consistant d'un soin anti-vieillissement, en particulier pour prévenir, corriger ou ralentir les effets du vieillissement de la peau; et d'un soin de revitalisation de la peau. Ses effets pour lutter contre le vieillissement de la peau comprennent principalement la lutte contre le vieillissement chronologique ou actinique de la peau.

Dans le cadre de l'invention, il a pu être mis en évidence un effet de synergie résultant de l'association du premier ingrédient cosmétiquement actif comprenant un extrait de sarment de vigne et du deuxième ingrédient cosmétiquement actif comprenant un composant de type ectoine.

L'extrait de sarment de vigne est, dans le cadre de l'invention, tout extrait de sarment de vigne récolté sur un vignoble. On peut utiliser des sarments de vigne de tout type de cépage, en particulier de cépage Merlot, de cépage Cabernet Franc, de cépage Gamay, de cépage Sirah, de cépage Sémillon, de cépage Sauvignon. Un sarment de vigne préféré selon l'invention est un sarment de vigne de cépage Sémillon et/ou de cépage Sauvignon.

Selon un mode de réalisation particulier, on utilise des sarments de vigne séchés. On entend par sarments de vigne séchés des sarments qui ont subi une étape de séchage pour présenter avantageusement un taux d'humidité inférieur à 20 %, de préférence inférieur à 5 % en poids.

Selon une variante de réalisation particulière, on réalise le séchage des sarments, soit par voie à l'air libre dans un endroit sec, soit par séchage dans une étuve à une température modérée, par exemple n'excédant pas environ 40 °C, jusqu'à obtention dans chaque cas d'un taux d'humidité inférieur à 20%, de préférence inférieur à 5% en poids.

Par exemple on peut réaliser le séchage par voie naturelle, à l'air libre, dans un endroit sec, selon le procédé de séchage préconisé dans le document précédent WO 01/03713, et comprenant en particulier une période de temps de séchage d'au moins deux mois, de préférence d'au moins quatre mois.

Ce séchage naturel à l'air libre favorise l'extraction ultérieure des ingrédients actifs, et notamment favorise la formation d'oligomères de resvératrol.

L'extrait de sarment de vigne utilisé dans le cadre de l'invention est avantageusement obtenu à l'aide d'un procédé qui permet d'aboutir à un extrait riche en molécules polyphénoliques, et en particulier en oligomères du resvératrol, qui sont recherchées dans le cadre de l'invention pour l'obtention d'un effet cosmétique.

Selon un mode de réalisation actuellement préféré de l'invention, la procédure d'extraction comprend principalement les étapes suivantes:

a) récolte des sarments de vigne sur le vignoble ;

b) séchage pendant une durée minimale de 4 mois ;

c) au moins une étape d'extraction avec un solvant organique polaire, par exemple alcool inférieur en $C_1$-$C_6$ tel que méthanol, éthanol, propanol, isopropanol, butanol, pentanol, hexanol, pur ou en mélange avec de l'eau ou une solution aqueuse ; une cétone, en particulier l'acétone, ou d'un mélange d'une cétone avec de l'eau, en obtenant ainsi un premier extrait de solvant polaire;

d) au moins un lavage dudit premier extrait de solvant polaire avec un solvant organique apolaire, tel que hexane, en obtenant ainsi un deuxième extrait, qui peut être utilisé tel quel ;

e) éventuellement, et dans la mesure où l'on recherche un extrait plus purifié, on réalise une purification sur colonne, en particulier une purification sur colonne de silice, réalisant la fixation des substances actives recherchées, principalement des substances polyphénoliques, et notamment les oligomères du resvératrol, puis leur élution sélective

avec un mélange éluant approprié, par exemple l'acétate d'éthyle/hexane, et notamment en proportion relative 80/20 v/v, en obtenant ainsi un troisième extrait hautement purifié, qui peut aussi être utilisé tel quel ;
f) éventuellement, on peut éliminer le solvant d'élution, notamment par évaporation, en obtenant une poudre de couleur beige, qui constitue l'extrait final purifié préféré de sarment de vigne selon l'invention.

Selon une variante de réalisation particulière de l'invention, cette poudre finale peut être re-solubilisée dans un mélange alcool/eau, par exemple à une proportion relative de 80/20 (v/v), en particulier un mélange de butylène glycol/eau 80/20 (v/v).

Cette poudre re-solubilisée dans ce mélange alcool/eau 80/20 (v/v) permet d'obtenir au moins environ 1%, avantageusement au moins 10% de viniférine, dimère du resvératrol, dans la solution finale.

En ce qui concerne le deuxième ingrédient cosmétiquement actif comprenant un composant de type ectoine, celui-ci est disponible dans le commerce. On peut par exemple utiliser un produit commercialisé par la société MERCK, par exemple sous la dénomination commerciale RONACARE® hydroine. Ce produit commercial de MERCK est particulièrement intéressant puisque la proportion relative ectoine/hydroxyectoine est sensiblement équivalente (ectoine 50-60 % en poids, hydroxyectoine 40.50 % en poids). Ce produit présente également une très bonne stabilité sans nécessiter l'ajout d'agent antioxydant ou de conservateur et est de qualité cosmétique.

D'autres buts, caractéristiques et avantages de l'invention apparaîtront clairement à la lumière de la description explicative, qui va suivre, faite en référence aux exemples ci-après selon l'invention, donnés simplement à titre d'illustration et qui ne sauraient donc en aucune façon limiter la portée de l'invention.

Dans les exemples, tous les pourcentages sont donnés en poids, la température est en degré Celsius, la pression est la pression atmosphérique, sauf indications contraires.

DESCRIPTION DES FIGURES

La figure 1 montre les résultats de l'essai de croissance cellulaire décrit à l'exemple 3, réalisé à l'aide du test XTT du commerce dénommé "Cell proliferation kit II" de chez BOEHRINGER, montrant la cinétique de croissance cellulaire respectivement pour le témoin sans traitement ; pour l'extrait de sarment de vigne 13 seul ; pour le produit "hydroine" (RONACARE® de Merck) ; et pour une combinaison de l'extrait 13 et de l'hydroine, réalisée à partir des mesures de croissance cellulaire à 6 heures, 9 heures, 15 heures et 18 heures, répertoriées en abscisses, et le nombre de cellules étant répertorié en ordonnées en terme de pourcentage de cellules par référence au nombre de cellules à 6 heures, considéré comme 100 % de cellules.

La figure 2 est une courbe dérivée de la figure 1, qui montre pour le temps à 18 heures de traitement des cellules, sous forme de bâtons, le nombre de cellules en pourcentage respectivement pour le contrôle sans traitement ; l'extrait I3; le produit "hydroine" (RONACARE® de Merck) ; et enfin l'association selon l'invention combinant l'extrait 13 et le produit hydroine, montrant l'effet de synergie de cette association.

La figure 3 représente les résultats du test de protection antî-radïcalaire réalisé à 4, en terme de pourcentage de protection donnée en ordonnées par rapport aux résultats sous forme de bâtonnets obtenus respectivement pour l'extrait 13 ; le produit "hydroine" (RONACARE® de Merck) ; et l'association de ces deux ingrédients actifs, répertoriés en abscisses, montrant la supériorité inattendue de protection de l'association vis-à-vis de chaque produit pris individuellement.

EXEMPLES DE L'INVENTION

EXEMPLE 1

Procédé de préparation d'un extrait de sarment de vigne selon l'invention dit I3

La procédure d'extraction comprend selon l'invention principalement les étapes suivantes :

a) récolte de 1 kg de sarments de vigne de cépage Sauvignon sur un vignoble situé au Sud-Est de Bordeaux, au mois de mars-avril;
b) on réalise le séchage des sarments de vigne recueillis à l'air, dans un local sec, pendant une durée minimale de 4 mois, jusqu'à un taux d'humidité inférieur à 5% en poids ;
c) pour réaliser l'extraction, on réalise un broyage des sarments de vigne séchés jusqu'à une granulométrie moyenne inférieure à 4 mm. On réalise au moins une étape d'extraction avec un solvant organique polaire, ici l'acétone, à la proportion en poids respective sarments broyés/solvant d'au moins 1/10, pendant une durée d'au moins 20 heures, à la température ambiante ou de l'ordre de 30°C; on procède à une filtration pour éliminer le résidu solide.
On évapore l'acétone de la solution d'acétone contenant l'extrait solubilisé, pour obtenir un premier extrait brut

sensiblement sec que l'on re-solubilise dans un mélange éthanol/eau dans une proportion 50/50 v/v, à raison de 225 ml environ pour cet extrait brut ayant une masse de l'ordre de 30g. La solution eau/éthanol contenant l'extrait solubilisé est maintenue à la température ambiante ou à une température comprise entre 25°C et 30°C sous agitation pendant une heure, et les surnageants sont séparés par filtration.

On obtient un extrait purifié à l'état solide, soit par élimination complète de l'éthanol par évaporation, soit après élimination partielle de l'éthanol par évaporation, en provoquant ainsi la précipitation de l'extrait purifié que l'on sèche de la manière habituelle en obtenant ainsi un extrait purifié sous la forme d'une poudre.

d) on effectue ensuite une nouvelle purification de l'extrait purifié ci-dessus en re-solubilisant la poudre obtenue dans un mélange acétone/eau dans une proportion relative 80/20, (v/v).

On ajoute de l'hexane jusqu'à obtenir une solution biphasique, la phase organique étant constituée essentiellement par l'hexane et la phase aqueuse comprenant essentiellement le mélange acétone/eau. On effectue ainsi une extraction sous forte agitation à la température ambiante pendant quelques minutes, par exemple environ 10mn.

On laisse reposer pour avoir une séparation des phases par décantation.

On récupère la phase aqueuse contenant l'extrait de sarment purifié recherché.

e) on réalise une évaporation des solvants de manière à obtenir un premier extrait de sarment purifié recherché qui peut être utilisé tel quel dénommé produit I1 de l'invention.

f) on réalise avantageusement une sur colonne de silice, réalisant la fixation des substances actives recherchées, principalement de substances et notamment les oligomères du resvératrol. On utilise dans cet exemple, comme colonne de silice, une colonne remplie avec de la silice de type 60 de Merck. On procède ensuite à une élution sélective des substances polyphénoliques fixées sur la silice à l'aide d'un solvant d'élution comprenant un mélange d'acétate d'éthyl/hexane en proportion relative 80/20, v/v, à raison de 1 litre de solvant par gramme d'extrait sec introduit dans la colonne ;

g) on élimine le solvant d'élution, notamment par évaporation, en obtenant ainsi une poudre de couleur beige, qui constitue un deuxième extrait final purifié de sarments de vigne selon l'invention, dit I2 ;

h) avantageusement, cette poudre finale I2 peut être re-solubilisée dans un mélange alcool/eau, ici butylène glycol/eau, à une proportion de 80/20 (v/v) pour faciliter son emploi dans une composition, notamment cosmétique.

Une analyse de cette poudre re-solubilisée dite produit ou extrait 13 selon l'invention, montre qu'elle contient environ 68 % d'oligomères de resvératrol, dont environ 24 % en viniférine.

EXEMPLE 2

Composition selon l'invention

On prépare une composition comprenant 50 % en poids d'extrait I3 obtenu à l'exemple 1 contenant environ 68 % d'oligomères du resvératrol, et environ 50 % de composants de type ectoine obtenus dans le commerce, à savoir le produit RONACARE® hydroine de la société MERCK.

Cette composition peut être diluée à diverses proportions afin de réaliser les essais, objets des exemples suivants.

EXEMPLE 3

Essais de croissance cellulaire

A - Modèle de croissance cellulaire

La composition de l'exemple 2 va être testée pour déterminer son influence sur la croissance cellulaire à l'aide du test XTT disponible dans le commerce dénommé "Cell proliferation kit II" de chez BOEHRINGER.

Selon ce modèle, le sel de Tétrazolium est transformé en Formazan, qui est un composé orange détecté à 450 nanomètres par un spectrofluoromètre, par exemple disponible dans le commerce sous la marque TECAN, par les déshydrogénases localisées dans les chaînes respiratoires mitochondriales. Ainsi, seules les cellules vivantes sont capables de former le Formazan.

La réaction est la suivante

Déshydrogénase mitochondriale + $e^-$

Sel de tetrazolium → Formazan

Les cellules sont traitées durant une période de 24 heures avec la composition selon l'invention de l'exemple 2, mais diluée à une concentration de 1,56 µg/ml pour chacun des deux ingrédients actifs (soit 1,56 µg/ml de la composition de l'exemple 2 et 1,56 µg/ml de RONACARE® de Merck, soit à titre comparatif avec une solution de composition de l'exemple 2 ou du produit RONACARE® hydroine seule, la concentration étant de 3,52 µg/ml de manière à avoir la même concentration totale.
On a réalisé des mesures avec le test XTT toutes les trois heures afin de suivre une cinétique de croissance cellulaire.
Il est à noter que le premier temps pris en considération, à savoir 6 heures après le début de est considéré comme 100 % de cellules. Il n'a pas été de prendre T0 ou T3, car les écarts type étaient trop importants, ceci étant probablement dû à la perturbation liée aux changements de milieu.

B - Protocole de traitement

Jour J0

Au jour JO, on réalise l'ensemencement des kératinocytes humains transformés, immortalisés, de type HaCaT bien connus de l'homme de l'art (voir http://cat.inist.fr/?aModele=afficheN&cpsidt=15688132 qui répertorie l'article de POZZI et al. dans Journal of Endocrinal. Invest. 2004, vol 27, N°2, p 147-149), dans une microplaque de 96 puits à raison de 10 000 cellules par puits.

Jour J24

A jour J24, on débute les traitements avec les produits prévus précités à la concentration indiquée, pour suivre la croissance cellulaire.

C - Observations des effets du traitement à 3 heures = T3, 6 heures = T6, 9 heures = T9, 15 heures = T15 et 18 heures = T18

A chaque étape de traitement indiqué dans le titre ci-dessus, on réalise l'élimination du milieu de culture (KSFM complémenté de chez Gibco).
On traite les cellules avec soit la composition de l'exemple 2 selon l'invention, soit l'extrait I3 seul, soit le produit hydroine commercial RONACARE® de Merck.
Pour cela, à partir d'une solution mère à 5 % dans le DMSO (diméthylsulfoxyde), on réalise au de façon non stérile une dilution respectivement au 1/16 et 1/32 à partir de la solution mère dans le DMSO pour obtenir pour le cas de la présence d'un seul produit, 3,12 mg/ml et dans le cas du mélange pour chacun d'entre eux, 1,56 mg/ml.
Puis, on réalise ensuite sous la hotte des dilutions au 1/1000 dans le milieu de culture, afin d'obtenir une concentration finale de respectivement 3,12 µg/ml pour le mélange et 1,56 µg/ml pour chaque ingrédient.
Ainsi, pour le test réalisé, les traitements ont été de :

- 3,12 µg/ml pour l'extrait I3 de l'exemple 1 ;
- 3,12 µg/ml pour le produit hydroine RONACARE® de MERCK ;
- la composition de l'exemple 2 de l'invention comprenant le mélange I3 de l'exemple 1 à 1,56 µg/ml avec le produit hydroine de RONACARE® de MERCK à 1,56 µg/ml.

D - Révélation du test

Pour révéler le test, on enlève le milieu de culture en retournant la microplaque.
On rince les cellules une fois : pour 200 µL/puits, le rinçage des cellules est réalisé avec du PBS à 37°C.
On ajoute 100 µL/puits de solution XTT à 0,2 mg/ml (dilution à 1/5 de la solution mère à 1 mg/ml), préparée extemporanément en milieu K-SFM complémenté.
On prépare un blanc sans cellule.

On enveloppe la microplaque dans du papier aluminium et on incube 3 heures à 37°C dans une étuve avec 5 % de $CO_2$.
A la fin de l'incubation de 3 heures, on réalise la lecture des densités optiques (DO) au spectrophotomètre disponible dans le commerce Tecan, à 450 nm.
L'expression des résultats obtenus est donnée ci-après

E - Expression des résultats

On considère le temps de traitement après 6 heures = T6, comme 100 % de cellules dans les puits.
Le pourcentage de viabilité est donné par la formule suivante :

$$\% \text{ de viabilité} = (\text{DO} \times 100) / (\text{DO Témoin-100})$$

RESULTATS

Les résultats sont exprimés au tableau I ci-après :

Tableau I

| Temps de traitement à J24 | % viabilité sans traitement | % viabilité avec 13 de l'exemple 1 | % viabilité avec hydroine RONACARE® | % viabilité avec la composition de l'invention de l'exemple 2 |
|---|---|---|---|---|
| 6 heures | 100 | 100 | 100 | 100 |
| 9 heures | 106 | 108 | 108 | 113 |
| 15 heures | 110 | 112 | 111 | 127 |
| 18 heures | 126 | 130 | 133 | 142 |

Les résultats obtenus sont donnés sous forme de courbe à la figure 1.
On observe à partir de ces résultats que pour les cellules traitées par la composition selon l'invention de l'exemple 2 comprenant une association du produit I3 de l'invention contenant une majorité d'oligomères du resvératrol, avec un produit de type hydroine, on obtient une croissance plus soutenue par rapport aux cellules non traitées, ou bien uniquement traitées par le produit I3 de l'exemple 1, ou le produit de type hydroine RONACARE® de Merck.
Il faut toutefois noter que le produit I3 de l'exemple 1 et le produit type hydroine présentent individuellement une activité faible.
Par ailleurs, grâce à la composition selon l'invention, la dynamique de croissance cellulaire est bien meilleure, le point d'arrivée à 18 heures étant le même.
On a représenté à la figure 2, pour le temps de traitement de 18 heures, la comparaison des résultats, qui montre bien une synergie d'action de l'association selon l'invention, tout à fait inattendue pour un homme de l'art.

EXEMPLE 4

Test de protection anti-radicalaire

Modèle : Test 3D (AES laboratoire)

Ce test permet la mise en évidence d'effet protecteur de l'ADN par une molécule, vis à vis des radicaux hydroxyles générés par l'eau oxygénée.
Pour caractériser une molécule ou un mélange présentant des propriétés anti-oxydantes ou anti-radicalaires, et donc protéger une cellule humaine des dommages oxydatifs dus aux espèces oxygénées réactives (EOR), 3 critères, établis par Barry Halliwell doivent être pris en compte :

- la molécule doit faire la preuve qu'elle est active dans la cellule, un test purement chimique le laissera supposer mais ne le démontrera pas ;

- la molécule doit être efficace vis-à-vis d'un oxydant biologique, c'est-à-dire un oxydant présent dans les cellules. Certains antioxydants ont une activité protectrice spécifique contre certains oxydants ;
- les concentrations d'oxydant utilisées doivent être compatibles avec celles utilisables *in vivo.*

Ces remarques de Barry Halliwell, spécialiste des oxydants, sont extraites de l'article paru en 1995 dans la revue Biochemical Pharmacology (volume 49, 1341-1348) intitulé "Antioxidant characterization - Methodology and Mechanism ".

Le *TEST 3D* permet donc la mise en évidence d'effet protecteur de l'ADN par une molécule, vis-à-vis des radicaux hydroxyles générés par l'eau oxygénée en présence de FeCl2.

De plus, le *TEST 3D* sur cellules répond parfaitement aux critères de Barry Halliwell.

Principe:

S.F.R.I. et l'équipe de Toxico-Résistance, I.P.B.S. CNRS de Toulouse, ont développé un système de détection de dommages *(TEST 3D)* sur de l'ADN capté sur microplaque (Analytical Biochemistry, 1995, 232, 37-42).

L'ADN est adsorbé sur des puits sensibilisés, puis incubé avec des agents oxydants (eau oxygénée + fer)). Les dommages engendrés sont reconnus puis réparés par les complexes protéiques spécifiques présents dans les extraits cellulaires purifiés d'origine humaine. La réparation des lésions implique une phase d'excision des lésions puis une resynthèse du fragment d'ADN ou des bases excisées.

Au cours de l'étape de synthèse réparatrice, des nucléotides modifiés (dUTP couplé à une biotine) sont incorporés dans l'ADN. Ces nucléotides biotinylés sont ensuite reconnus par une molécule d'avidine couplée à la peroxydase . Un substrat chimiluminescent de la peroxydase est ensuite ajouté et le signal émis est mesuré par un luminomètre (spectrafluorplus, Tecan). L'intensité du signal mesuré, désigné ci-après RLU, est fonction du nombre de lésions réparées sur l'ADN. Un effet dose est observé dans la limite de 1 à 15 lésions pour 6 kilobases et ce, pour la plupart des lésions.

Ce système est susceptible de réparer tous les types de lésions puisque les différentes voies de la réparation des lésions de l'ADN (NER et BER) sont présentes et actives dans les extraits cellulaires préparés et utilisés pour cette étude. Les dommages oxydatifs sont donc reconnus dans ce système.

MATERIEL ET METHODES

1. Matériel

Les réactifs utilisés ont été décrits dans l'article Analytical Biochemistry, 1995, 232, 37-42.

Le signal chimiluminescent est détecté à l'aide d'un luminomètre spectrafluorplus (Tecan).

2. Méthodes *TEST 3D in vitro*

2.1 Dilutions des échantillons à tester

Les échantillons d'extrait de sarment I3 et d'hydroine sont mis en solution dans du méthanol à la concentration stock de 50 mg/ml. Les concentrations étudiées sont préparées par dilutions successives à partir de la solution stock dans de l'eau ultrapure. Toutes les dilutions sont préparées 2 fois plus concentrées, afin d'obtenir les concentrations voulues après addition d'un volume d'une solution oxydante d'eau oxygénée + FeCl2.

Afin de vérifier la spécificité de la diminution du signal de réparation, les mêmes dilutions (1X) ont été incubées dans les mêmes conditions sur de l'ADN lésé par eau oxygénée + FeCl2. Une dilution ne présentera un effet protecteur que si l'on note un abaissement du signal de réparation en présence d'oxydant, et une absence de modification significative du signal sur l'ADN préalablement lésé. Une inhibition du signal de réparation sur l'ADN lésé, peut, par exemple, être expliquée par une interaction directe de l'échantillon avec l'ADN ce qui aurait pour effet de bloquer l'accès des lésions aux complexes protéiques de réparation.

2.2 Adsorption de l'ADN cible dans les puits de microplaque

De l'ADN plasmidique (pBS) ultra purifié (forme super enroulée majoritaire) est mis en contact avec les puits sensibilisés pendant 30 minutes à 30°C sous agitation légère. Dans ces conditions, l'adsorption de l'ADN est quantitative.

Un contrôle positif de réparation consistant en un ADN plasmidique pré-endommagé avec H2O2 + FeCl2 est ajouté.

2.3 Recherche d'un effet protecteur des échantillons vis-à-vis d'espèces oxygénées réactives

Le radical hydroxyle OH° est produit par réaction de Fenton (eau oxygénée + FeCl2).

Réaction de Fenton : Réalisée à partir de l'addition d'un mélange $H_2O_2$ + $FeCl_2$

$$H_2O_2 + Fe^{2+} \longrightarrow Fe^{3+} + OH^- + \mathbf{OH°}$$

Ces radicaux, puissants électrophiles, possèdent une durée de vie très courte (de l'ordre de $10^{-9}$ seconde). Ils réagissent donc très rapidement avec les bases de l'ADN et produisent divers dommages tels que des modifications ou des pertes de bases. Ces lésions très génotoxiques sont reconnues par le système de réparation.
L'eau oxygénée est préparée à 4mM dans de l'eau ultra pure (qualité MilliQ de Millipore). Le FeCl2 est préparé à 2µM dans de l'eau ultra pure. Les deux solutions sont diluées volume à volume extemporanément.
Cette solution est mélangée à volume égal extemporanément avec les différentes dilutions des échantillons à tester. 50µl du mélange sont ajoutés dans les puits contenant l'ADN plasmidique adsorbé. L'ensemble est ensuite incubé 30 minutes à 30°C sous agitation douce.
Ensuite, une étape de rinçage est effectuée afin de ne conserver que l'ADN plus ou moins lésé par l'oxydant.
Puis, l'étape de réparation des lésions est par les complexes spécifiques de réparation. Au cours de la phase de resynthèse du brin d'ADN excisé, un nucléotide marqué à la biotine est incorporé dans l'ADN (incubation 3 heures à 30°C sans agitation).
Ensuite, une étape de rinçage est effectuée afin d'éliminer la biotine non incorporée dans l'ADN.
Vient ensuite une étape de reconnaissance de la biotine par une molécule d'avidine couplée à une molécule de peroxydase (15 minutes à 30°C avec agitation douce).
Puis, une étape de rinçage est de nouveau effectuée afin d'éliminer l'avidine couplée à une molécule de peroxydase non fixée sur la biotine.
Et enfin, une étape de révélation de la réaction de réparation est réalisée par addition d'un substrat chimiluminescent de la peroxydase (5 minutes à 30°C sous agitation douce) puis lecture de luminescence.

INTERPRETATION DES RESULTATS

Pour une meilleure visualisation des résultats, ceux-ci sont exprimés en pourcentage de protection ou pourcentage d'inhibition de la formation de dommages oxydatifs sur l'ADN. La valeur 0% correspond au signal de la réparation de l'ADN lésé par l'oxydant seul.
Le pourcentage de protection en présence de EOR est calculé comme la baisse relative de l'effet lésionnel dû aux radicaux OH° ou $^1O_2$, soit :

$$\frac{[\text{RLU oxydant seul}] - [\text{RLU (oxydant + échantillon)}]}{[\text{RLU oxydant seul}]} \text{ X } 100$$

Une inhibition non spécifique (en absence de EOR) du signal de réparation être parfois observée. Ceci peut être dû à une interaction directe du composé avec l'ADN (désorption de l'ADN du puits, association non spécifique avec l'ADN qui va masquer les lésions aux protéines de réparation...). Un contrôle consistant à incuber les agents testés avec de l'ADN préalablement lésé est donc ajouté. Une diminution du signal dans cette condition reflète une inhibition non spécifique du composé, indépendante de ses possibles propriétés anti-radicalaires.
Une inhibition de la réparation en présence de EOR peut être la conséquence :

- d'une réelle protection
- d'une diminution de l'efficacité de la réparation d'un ADN endommagé due à une interaction directe de la molécule

à tester avec l'ADN ou la microplaque traitée
- de la survenue simultanée de ces 2 phénomènes.

L'inhibition non spécifique est donc évaluée. Celle-ci est calculée à partir des résultats obtenus sur les échantillons incubés sur de l'ADN préalablement lésé.
L'inhibition spécifique ou protection spécifique est égale à la différence entre l'inhibition en présence de EOR et l'inhibition non spécifique. Elle est le reflet de l'activité anti-radicalaire due à la molécule seulement.

TABLEAU II

| Test de protection anti-radicalaire | |
|---|---|
| Echantillons | % protection |
| Témoin solvant | - |
| I3 | 43 |
| Hydroine RONACARE® | 47 |
| I3 + hydroine RONACARE® (invention) | 62 |

Les échantillons d'extrait de sarment 13 et d'hydroine présentent une activité protectrice vis à vis des radicaux libres. Lors du traitement avec la composition de l'invention, on augmente d'environ 40% l'efficacité protectrice.
Il existe donc bien une synergie entre les deux molécules.
Les doses recommandées sont de 0.1% à 10% du mélange 1/1 extrait de sarment / composant hydroine.
Comme il résulte du tableau II et de la figure 3 annexée, il apparaît que chaque échantillon de produit respectivement 13 et de produit hydroine présente une activité protectrice vis-à-vis des radicaux libres.
Par contre, grâce à la composition selon l'invention combinant le produit I3 et le produit hydroine, on obtient une augmentation d'environ 40 % de l'efficacité protectrice démontrant un effet de synergie entre les deux molécules tout à fait inattendu pour un homme de l'art.

EXEMPLE 5

Mise en évidence de l'activité anti-radicalaire de l'association selon l'invention

Les espèces radicalaires d'oxygène sont produites dans la peau par différents facteurs agressifs tels que le rayonnement UV, les stress et les réactions inflammatoires.
Ces molécules oxydantes attaquent les constituants de la peau notamment les lipides, les protéines, les polysaccharides et l'ADN cellulaire, perturbant ainsi les fonctions biologiques.
Le test utilisé ici pour mettre en évidence l'effet protecteur de l'association des extraits de sarment et des composants de type ectoine, selon l'invention, après son application sur la peau est basé sur celui décrit dans la publication intitulée "A new method for assessing, in vivo in human subjects, the basal or UV-induced peroxydation of the stratum corneum. Application to test the efficacy of free-radical-scavenging products", de P. Girard, L. Lempereur, P. Buche et L. Violin, dans la revue Curr. Probl. Dermatol., 1998, 26, 99-107.
Le principe de ce test est basé sur la réaction des radicaux libres d'oxygène présents à la surface de la peau avec un marqueur constitué par de la DCF(H)-DA (2'7' dichlorofluorescein diacetate) qui conduit à un produit fluorescent, noté DCF*, dont la fluorescence est mesurée au fluorimètre. La mesure de cette fluorescence permet de quantifier la présence de radicaux libres et par conséquence le degré d'activité d'un produit protecteur.
Trente volontaires sains de type caucasien et de sexe féminin ont été sélectionnés.
Le produit à tester est appliqué sur une zone de la partie interne d'un avant-bras à raison de 2 µl/cm2 par application. On effectue quatre applications espacées de 2 h chacune.
La personne testée est autorisée par la suite à employer ses produits nettoyants habituels à l'exclusion de tout produit de soins cutanés.
24 h après la première application du produit à tester, on effectue un prélèvement au moyen de disques adhésifs transparents standard, disponibles dans le commerce sous la marque D-Squames®.
La quantité de cornéocytes ainsi prélevée est évaluée par mesure de transmission de lumière infrafrouge au travers de D-Squames®, avec un dispositif du commerce dénommé Squameter, afin de pouvoir tenir compte du nombre de squames dans l'analyse statistique de résultats de mesure.

On met ensuite en incubation les différents disques D-Squames® dans une solution tampon-phosphate 66mM à pH 7,4 pendant 32 h à 37°, contenant du DCF*. La fluorescence de la solution dans chaque cas après incubation est quantifiée au de la marque Fluoroskan Ascent (Labsystems).

Les produits suivants ont été testés:

Produit 1 (de l'invention): émulsion (crème) contenant, en poids, 1% d'extrait I3 et 1% d'hydroine RONACARE® de la société MERCK, ci-après "hydroine",
Produit 2 (de l'invention): lotion contenant 1% d'extrait I3 et 1,5% d'hydroïne,
Produit 3: émulsion contenant des polyphénols de pépins de raisin,
Produit 4: émulsion contenant 0,5% d'idébénone, agent anti-oxydant bien connu,
Produit 5: émulsion contenant 1% d'idébénone

Les résultats de mesure de fluorescence sont reportés au tableau III ci-dessous :

TABLEAU III

| | Fluorescence | Comparaison au TNT (%) |
|---|---|---|
| Produit 1 *(de l'invention)* | 106.6 | -19% |
| Produit 2 *(de l'invention)* | 101.0 | -23% |
| Produit 3 | 121.1 | NS |
| Produit 4 | 126.5 | NS |
| Produit 5 | 114.7 | -13% |
| Témoin *(sujet non traité) ("TNT")* | 131.7 | |
| *S (<0.01)* | | |

Ainsi, la fluorescence étant directement corrélée à la quantité de radicaux libres présents, il apparaît clairement d'après les résultats ci-dessus que la quantité de radicaux libres est très significativement plus faible en présence des produits de l'invention. En conséquence, ceux-ci présentent donc une meilleure action anti-radicalaire, in vivo.

On donnera ci-après divers exemples de formulation cosmétique de la composition selon l'invention, dont la formulation avec les différents ingrédients est réalisée d'une manière classique par l'homme de l'art,

EXEMPLE 6

| Composition cosmétique sous forme de crème selon l'invention | | |
|---|---|---|
| Steareth-21 (Brij 721) | 2,5 | % |
| Glyceryl stearate (Tegin) | 1,1 | |
| Alcool stéarylique | 5 | |
| Tricaprat / caprylate glycérol | 11,5 | |
| Butylene glycol | 3 | |
| Glycérine | 2 | |
| Conservateur | 0,5 | |
| Concentré de parfum | 0,5 | |
| Eau | 62,4 | |
| Composition de l'exemple 2 | 5 | |
| Methoxycinnamate d'octyle | 7,5 | |

EXEMPLE 7

| Composition cosmétique sous forme de gel | | |
|---|---|---|
| Glycol | 3 | % |
| Polymère d'AMPS (Sepigel 305) | 3 | |
| Huile de ricin hydrogénée (Crémophor CO-60) | 2 | |
| Polyéthylène glycol | 1,5 | |
| Conservateur | 0,5 | |
| Concentré de parfum | 0,3 | |
| Eau | 85,7 | |
| Composition de l'exemple 2 | 3 | |
| Benzophénone 4 | 1 | |

EXEMPLE 8

| Composition cosmétique sous forme de lotion | | |
|---|---|---|
| Butylène glycol | 3 | % |
| EDTA | 0,1 | |
| Solubilisant | 1 | |
| Concentré de parfum | 0,3 | |
| Alcool | 5 | |
| Eau | 80,47 | |
| Composition de l'exemple 2 | 10 | |
| Benzophénone 4 | 0,13 | |

## Revendications

1. Composition, **caractérisée en ce qu'**elle comprend une association d'un premier ingrédient cosmétiquement actif comprenant un extrait de sarment séché de vigne et d'un deuxième ingrédient cosmétiquement actif comprenant un composant de type ectoine.

2. Composition selon la revendication 1, **caractérisée en ce que** le composant de type ectoine est un acide (S)-1,4,5,6-tetrahydro-4-pyrimidine carboxylique, substitué ou non par au moins un radical alkyle inférieur en C1-C6, notamment en position 2, et/ou par au moins un groupe hydroxy ou methoxy, notamment en position 5, ainsi que ses sels et esters cosmétiquement acceptables.

3. Composition selon la revendication 1 ou 2, **caractérisée en ce que** le deuxième ingrédient est choisi parmi l'ectoine et l'hydroxyectoine ainsi que leurs sels ou esters cosmétiquement acceptables.

4. Composition selon l'une des revendications 1 à 3, **caractérisée en ce** ledit deuxième ingrédient est un mélange en proportions sensiblement égales en poids d'ectoine et d'hydroxyectoine.

5. Composition selon l'une des revendications 1 à 4, **caractérisée en ce qu'**elle comprend de 0,1 % à 20 % en poids, de préférence de 0,1 % à 10 % en poids, dudit premier ingrédient, et de 0,1 % à 20 % en poids, de préférence de 0,1 % à 10 % en poids, dudit deuxième ingrédient, le total de ces deux ingrédients étant toutefois avantageusement inférieur ou égal à 20 % en poids, de préférence inférieur à 10% en poids.

6. Composition selon l'une des revendications 1 à 5, **caractérisée en ce que** la proportion respective en poids entre ledit premier ingrédient et ledit deuxième ingrédient est comprise entre 1/10 et 10/1, en particulier elle est d'environ 1/1.

7. Composition cosmétique à application topique sur la peau, **caractérisée en ce qu'**elle comprend une composition telle que revendiquée à l'une quelconque des revendications précédentes, associée à un excipient cosmétiquement

acceptable.

**8.** Composition selon la revendication 7, **caractérisée en ce qu'**elle comprend au moins un autre ingrédient cosmétiquement actif, en particulier un autre ingrédient cosmétiquement actif ayant une activité anti-vieillissement, en particulier pour prévenir, corriger ou ralentir les effets du vieillissement de la peau; par exemple la vitamine A, vitamine E, vitamine C; ou réalisant un soin de revitalisation de la peau, par exemple un madécassoside; ou un ingrédient cosmétiquement actif, ayant un effet hydratant, par exemple un ecdystéroïde ou un extrait de plante en contenant, tel qu'un extrait de Ajuga turkestanica; ou un ingrédient cosmétiquement actif de protection contre les rayonnements actiniques, par exemple un filtre solaire physique ou chimique cosmétiquement acceptable, par exemple un méthoxycinnamate.

**9.** Procédé de soin cosmétique, **caractérisé en ce qu'**il comprend l'application topique, sur la peau d'une personne voulant réaliser un soin cosmétique, d'une composition telle que définie à l'une quelconque des revendications 1 à 6, ou d'une composition cosmétique telle que définie à la revendication 7 ou 8.

**10.** Procédé selon la revendication 9, **caractérisé en ce qu'**il s'agit d'un soin cosmétique choisi parmi le groupe consistant d'un soin anti-vieillissement, en particulier pour prévenir, corriger ou ralentir les effets du vieillissement de la peau; et d'un soin de revitalisation de la peau.

## Claims

**1.** A composition, which comprises an association of a first cosmetically active ingredient comprising a dried vine shoot extract, and a second cosmetically active ingredient of the ectoine type.

**2.** The composition as claimed in claim 1, wherein the component of the ectoine type is an (S)-1,4,5,6-tetrahydropyrimidine-4-carboxylic acid which is unsubstituted or substituted by at least one C1-C6 lower alkyl radical, especially in the 2-position, and/or by at least one hydroxyl or methoxy group, especially in the 5-position, and its cosmetically acceptable salts and esters.

**3.** The composition as claimed in claim 1 or 2, wherein the second ingredient is selected from ectoine and hydroxyectoine and their cosmetically acceptable salts or esters.

**4.** The composition as claimed in one of claims 1 to 3, wherein the second ingredient is a mixture of ectoine and hydroxyectoine in substantially equal proportions by weight.

**5.** The composition as claimed in one of claims 1 to 4, which comprises from 0.1% to 20% by weight, preferably from 0.1% to 10% by weight, of said first ingredient and from 0.1% to 20% by weight, preferably from 0.1% to 10% by weight, of said second ingredient, but the total of these two ingredients is advantageously less than or equal to 20% by weight, preferably less than 10% by weight.

**6.** The composition as claimed in one of claims 1 to 5, wherein the respective proportions by weight of said first ingredient and said second ingredient are between 1/10 and 10/1, particularly about 1/1.

**7.** A cosmetic composition for topical application to the skin which comprises a composition as claimed in any one of the claims, associated witch a cosmetically acceptable excipient.

**8.** The composition as claimed in claim 7, which comprises at least one other cosmetically active ingredient, especially another cosmetically active ingredient which has anti-ageing activity, particularly for preventing, correcting or slowing down the effects of skin ageing, e.g. vitamin A, vitamin E or vitamin C; or a cosmetically active ingredient which effects skin revitalization care, e.g. a madecassoside; or a cosmetically active ingredient which has a moisturizing effect, e.g. an ecdysteroid or a plant extract containing it, such as an extract of Ajuga turkestanica; or a cosmetically active ingredient for protecting against actinic radiation, e.g. a cosmetically acceptable physical or chemical sunscreen such as a methoxycinnamate.

**9.** A method of cosmetic care, which comprises the topical application, to the skin of a person who wishes to effect cosmetic care, of a composition as defined in any one of claims 1 to 6 or a cosmetic composition as defined in claim 7 or 8.

10. The method as claimed in claim 9, wherein the cosmetic care is selected from the group comprising an anti-ageing care, particularly for preventing, correcting or slowing down the effects of skin ageing, and a skin revitalization care.

## Patentansprüche

1. Zusammensetzung, **dadurch gekennzeichnet, daß** sie eine Verbindung aus einem kosmetisch aktiven ersten Inhaltsstoff, welcher einen Extrakt aus getrockneter Weinrebe enthält, und aus einem kosmetisch aktiven zweiten Inhaltsstoff, der einen ectoinartigen Bestandteil enthält, umfaßt.

2. Zusammensetzung nach Anspruch 1, **dadurch gekennzeichnet, daß** der ectoinartige Bestandteil eine (S)-1,4,5,6-Tetrahydropyrimidin-4-Carbonsäure, substituiert oder nicht durch wenigstens ein niederes C1- bis C6-Alkylradikal, insbesondere an Position 2, und/oder durch wenigstens eine Hydroxy- oder Methoxygruppe, insbesondere an Position 5, sowie deren kosmetisch akzeptable Salze und Ester ist.

3. Zusammensetzung nach Anspruch 1 oder 2, **dadurch gekennzeichnet, daß** der zweite Inhaltsstoff unter Ectoin und Hydroxyectoin sowie deren kosmetisch akzeptablen Salzen oder Estern ausgewählt ist.

4. Zusammensetzung nach einem der Ansprüche 1 bis 3, **dadurch gekennzeichnet, daß** der zweite Inhaltsstoff eine Mischung aus im wesentlichen gleichen Gewichtsanteilen Ectoin und Hydroxyectoin ist.

5. Zusammensetzung nach einem der Ansprüche 1 bis 4, **dadurch gekennzeichnet, daß** sie zwischen 0,1 und 20 Gew.-%, vorzugsweise zwischen 0,1 und 10 Gew.-% des ersten Inhaltsstoffes sowie zwischen 0,1 und 20 Gew.-%, vorzugsweise zwischen 0,1 und 10 Gew.-% des zweiten Inhaltsstoffes enthält, wobei die Summe dieser beiden Inhaltsstoffe jedoch vorteilhafterweise kleiner als oder gleich 20 Gew.-%, vorzugsweise kleiner als 10 Gew.-% ist.

6. Zusammensetzung nach einem der Ansprüche 1 bis 5, **dadurch gekennzeichnet, daß** das jeweilige Gewichtsverhältnis zwischen dem ersten Inhaltsstoff und dem zweiten Inhaltsstoff zwischen 1/10 und 10/1 liegt, insbesondere etwa 1/1 beträgt.

7. Kosmetische Zusammensetzung zur topischen Anwendung auf der Haut, **dadurch gekennzeichnet, daß** sie eine Zusammensetzung, wie sie in einem der vorhergehenden Ansprüche beansprucht ist, in Verbindung mit einem kosmetisch akzeptablen Bindemittel enthält.

8. Zusammensetzung nach Anspruch 7, **dadurch gekennzeichnet, daß** sie wenigstens einen weiteren kosmetisch aktiven Inhaltsstoff enthält, insbesondere einen weiteren kosmetisch aktiven Inhaltsstoff, der eine Anti-Aging-Aktivität aufweist, insbesondere zur Vorbeugung, Korrektur oder Verlangsamung der Wirkungen der Hautalterung; beispielsweise Vitamin A, Vitamin E, Vitamin C; oder der eine Pflege zur Revitalisierung der Haut bewirkt, zum Beispiel ein Madecassosid; oder einen kosmetisch aktiven Inhaltsstoff, der eine hydratisierende Wirkung hat, beispielsweise ein Ecdysteroid oder einen dieses enthaltenden Pflanzenextrakt, wie zum Beispiel einen Ajuga turkestanica Extrakt; oder einen kosmetisch aktiven Inhaltsstoff zum Schutz gegen die aktinischen Strahlungen, beispielsweise ein kosmetisch akzeptables physikalisches oder chemisches Sonnenfilter, zum Beispiel ein Methoxycinnamat.

9. Kosmetisches Pflegeverfahren, **dadurch gekennzeichnet, daß** es die topische Anwendung einer Zusammensetzung, wie sie in einem der Ansprüche 1 bis 6 definiert ist, oder einer kosmetischen Zusammensetzung, wie sie in Anspruch 7 oder 8 definiert ist, auf der Haut einer Person, die eine kosmetische Pflege betreiben möchte, umfaßt.

10. Verfahren nach Anspruch 9, **dadurch gekennzeichnet, daß** es sich um eine kosmetische Pflege ausgewählt aus der Gruppe umfassend eine Anti-Aging-Pflege, insbesondere zur Vorbeugung, Korrektur oder Verlangsamung der Wirkungen der Hautalterung, sowie um eine Pflege zur Revitalisierung der Haut handelt.

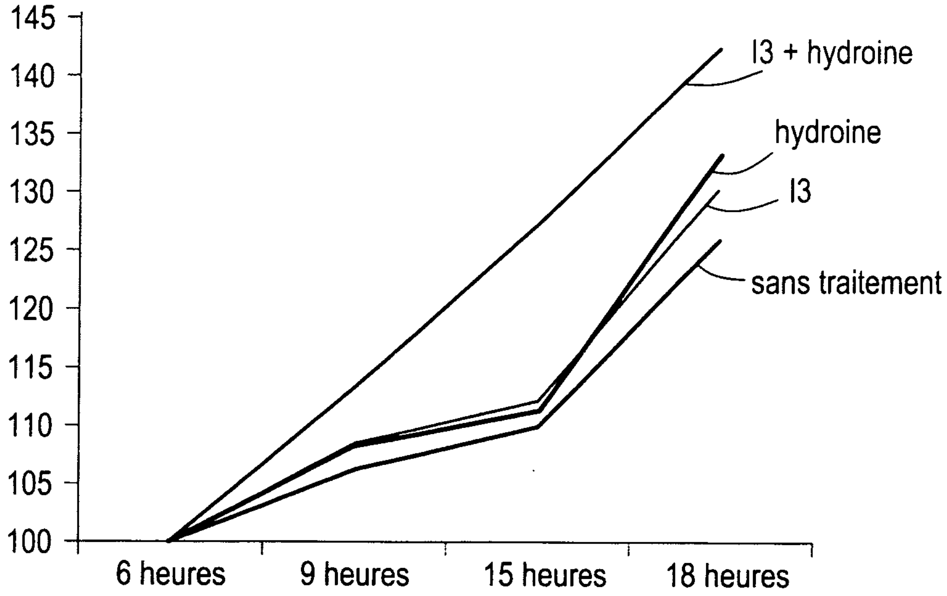


FIG.1

Temps 18 heures

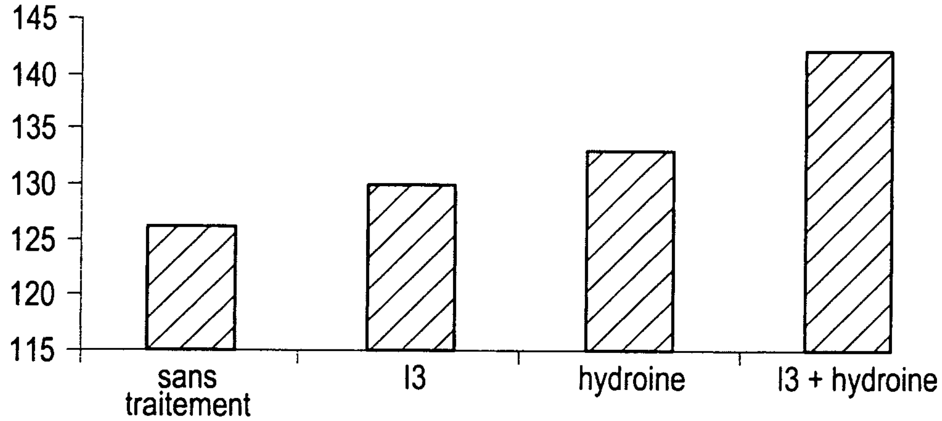


FIG.2

% protection

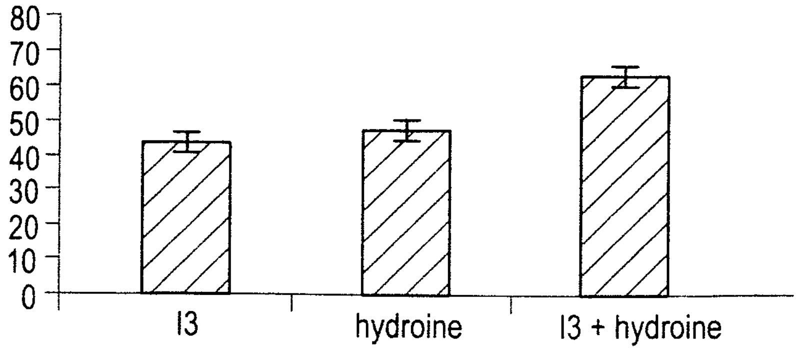


FIG.3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0103713 A


### Littérature non-brevet citée dans la description


- **POZZI et al.** *Journal of Endocrinal. Invest.,* 2004, vol. 27 (2), 147-149
- Antioxidant characterization - Methodology and Mechanism. *Biochemical Pharmacology,* vol. 49, 1341-1348
- *Analytical Biochemistry,* 1995, vol. 232, 37-42
- **P. GIRARD ; L. LEMPEREUR ; P. BUCHE ; L. VIOLIN.** A new method for assessing, in vivo in human subjects, the basal or UV-induced peroxydation of the stratum corneum. Application to test the efficacy of free-radical-scavenging products. *Curr. Probl. Dermatol.,* 1998, vol. 26, 99-107